(19) **Europäisches Patentamt / European Patent Office / Office européen des brevets**

(11) **EP 1 424 910 B1**

(12) **EUROPÄISCHE PATENTSCHRIFT**

(45) Veröffentlichungstag und Bekanntmachung des Hinweises auf die Patenterteilung:
**25.04.2007   Patentblatt 2007/17**

(21) Anmeldenummer: **02767433.2**

(22) Anmeldetag: **27.08.2002**

(51) Int Cl.:
***A23N 1/00*** *(2006.01)*

(86) Internationale Anmeldenummer:
**PCT/EP2002/009529**

(87) Internationale Veröffentlichungsnummer:
**WO 2003/022080 (20.03.2003 Gazette 2003/12)**

(54) **ELEKTROPORATIONSREAKTOR ZUR KONTINUIERLICHEN PROZESSIERUNG VON STÜCKIGEN PRODUKTEN**

ELECTROPORATION REACTOR FOR CONTINUOUSLY PROCESSING PRODUCTS IN THE FORM OF PIECES

REACTEUR D'ELECTROPORATION PERMETTANT LE TRAITEMENT EN CONTINU DE PRODUITS EN MORCEAUX

(84) Benannte Vertragsstaaten:
**AT BE BG CH CY CZ DE DK EE ES FI FR GB GR IE IT LI LU MC NL PT SE SK TR**

(30) Priorität: **10.09.2001   DE 10144479**

(43) Veröffentlichungstag der Anmeldung:
**09.06.2004   Patentblatt 2004/24**

(73) Patentinhaber:
• **Forschungszentrum Karlsruhe GmbH**
**76133 Karlsruhe (DE)**
• **Kea-Tec GmbH**
**68753 Waghäusel (DE)**

(72) Erfinder:
• **SCHULTHEISS, Christoph**
**76327 Pfinztal (DE)**
• **KERN, Martin**
**68753 Waghäusel (DE)**

(56) Entgegenhaltungen:
**US-A- 4 787 303**

• **DATABASE WPI Section Ch, Week 199048 Derwent Publications Ltd., London, GB; Class D14, AN 1990-359848 XP002223760 -& SU 1 336 293 A (AS MOLD APPLD PHYSI), 15. Mai 1990 (1990-05-15)**
• **DATABASE WPI Section Ch, Week 198115 Derwent Publications Ltd., London, GB; Class D13, AN 1981-26640D XP002223761 -& SU 751 387 A (AS MOLD APPL PHYS), 30. September 1980 (1980-09-30)**
• **DATABASE WPI Section Ch, Week 198217 Derwent Publications Ltd., London, GB; Class D14, AN 1982-34883E XP002223762 -& SU 843 931 A (AS MOLD APPLD PHYS), 7. Juli 1981 (1981-07-07)**
• **DATABASE WPI Section Ch, Week 198240 Derwent Publications Ltd., London, GB; Class D14, AN 1982-85577E XP002223763 -& SU 888 921 A (MOLD FOOD IND MIN), 15. Dezember 1981 (1981-12-15)**
• **DATABASE WPI Section Ch, Week 198134 Derwent Publications Ltd., London, GB; Class D14, AN 1981-61986D XP002224909 & SU 786 966 A (AS MOLD APPLD PHYS), 25. Dezember 1980 (1980-12-25)**

**EP 1 424 910 B1**

**Beschreibung**

Die Erfindung betrifft einen Elektroporationsreaktor zur großtechnischen kontinuierlichen Prozessierung von stückigen Produkten, dem Prozessgut, insbesondere Agrarprodukten, wie Zuckerrüben, Kartoffel, Gemüse, Obst, Heilpflanzen und auch tierischen Produkten, in einer Prozessflüssigkeit mit Hochspannungsimpulsen. Die Agrarprodukte liegen in wie bei der Ernte anfallenden ganzen Elementen als absiebbare Fraktion oder vorzerkleinert vor.

Das Verfahren zum Aufschluss des Zellmaterials mit gepulsten elektrischen Feldern (Hochspannungsentladungen) wird als Elektroporation bzw. Elektroplasmolyse bezeichnet.

Aus der Literatur sind Vorrichtungen bekannt, die zur Behandlung von Pflanzenzellen bzw. pumpbaren Lebensmitteln eingesetzt werden. Beispielsweise die folgenden:

> US 3,766,050 "Apparatus for the treatment of fluids or solutions by electric fields"; 1973-10-16. In ihr werden Reaktorbauformen mit unterschiedlich angeordneten Elektroden und dimensionierten Strömungskanälen beschrieben. Die Reaktoren sind ausschließlich für kleine Mengen und kleine Partikelgrößen einsetzbar.

US 4,723,483 "Electroplasmolyzer for processing vegetable stock"; 1988-02-09 bzw. FR 2 619 489 "Electroplasmolyzer for processing vegetable materials"; 1989-02-24. In diesen Literaturstellen wird ein runder bzw. rechteckiger Reaktorquerschnitt beschrieben, in dem Elektrodenpaare in unterschiedlichen Anordnungen installiert sind. Das Produkt wird durch Schwerkraft oder Pumpendruck durch den Reaktor gefördert.

US 5,031,521 "Electroplasmolyser for processing plant raw material"; 1991-07-16. Darin wird eine ähnliche Reaktorgeometrien wie US 4,723,483 beschrieben, die elektrische Energie wird jedoch durch Elektromagneten appliziert.

US 5,186,800 "Electroporation of prokaryotic cells"; 1993-02-16. Hier werden kleinste Laborreaktoren beschrieben, in denen kleine Produkt mengenweise mit Spannungsimpulsen behandelt werden. Die Reaktoren weisen keine bewegten Teile auf.
US 5,549,041 "Batch mode food treatment using pulsed electric fields"; 1996-08-27 Diese Schrift beschreibt kleine Reaktoren mit flächigen Elektroden zwischen denen zu behandelnde Suspensionen gepumpt werden.

Schließlich beschreibt die US 4,787,303 einen Elecktroportionsrechter, bei dem das Behandlungsgert kontinuierlich mittels eines Rotors durch dem Behandlungsraum gefördert wird.

Das Verfahren der Elektroporation wird zur Gewinnung von intrazellulären Substanzen eingesetzt. Dabei werden die wertbringenden Stoffe meist abgepresst bzw. über Extraktionsvorgänge gewonnen. Die Behandlung mit gepulsten elektrischen Feldern erfolgt in einer Prozessflüssigkeit, die zumeist Wasser mit geringer Leitfähigkeit ist.

Bekannte Vorrichtungen (Reaktoren) sind für pumpbare Lebensmittel und Suspensionen einsetzbar.

Sollen nun auch stückige Produkte mit 20 - 30 Sortenelementen, Stückgewichte 1 - 5 kg behandelt werden, ist eine Produktförderung durch bekannte Reaktoren nicht mehr möglich.

Die industrielle Prozessierung zu verarbeitenden Prozessgutes wie agrarisches Stückgut mit Hochspannungsimpulsen erfordert gegenüber den bisher bekannten Vorrichtungen zur Elektroporation einen hohen kontinuierlichen Massedurchsatz bei einer möglichst gleichmäßigen Einwirkung eines gepulsten elektrischen Feldes.

Die dabei auftretenden Schwierigkeiten sowie die Nachteile des Stands der Technik sind folgende:

- Produktdurchsatz
  Bei den in der Vorrichtung zu behandelnden Agrarprodukte sind oftmals hohe Stundendurchsätze erforderlich (z.B.: Zuckerindustrie 600 Mg Rüben/ h). Die Vorrichtung muss hohe Durchsätze bei geringster Produktschädigung ermöglichen.
- Produkttransport
  Zwischen dem zu behandelnden Produkt und der zur Pulsbehandlung erforderlichen Flüssigkeit besteht nur ein geringer Dichteunterschied. Dies führt dazu, dass aufgrund der geringen Sinkgeschwindigkeit bei selbstständigem Nachrutschen des Produkts keine ausreichend großen Produktdurchsätze möglich sind.
- Verstopfungsproblematik
  Aufgrund der unterschiedlichen geometrischen Formen der zu behandelnden Agrarprodukte ist ein erhöhte Anfälligkeit für Verstopfungen und Brückenbildung gegeben.
- Reaktorgeometrie, Verstopfung
  Aufgrund der zur Begrenzung des Energiebedarfs erforderlichen hohen Feldstärken (Elektrobepulsung) sind auch bei hohen Pulsspannungen keine sehr großen Reaktordurchmesser realisierbar. Kleine Reaktordurchmesser weisen eine hohe Verstopfungsneigung auf.
- Produktverlust
  Zur Vermeidung von Produktverlusten (Vorextraktion) und zur Begrenzung der in der Betriebsflüssigkeit akkumulierenden Elektrolyten ist die Behandlung unversehrter Produkte (ganze Rüben, Äpfel, Tomaten, Gurken u.s.w.) sinnvoll jedoch nicht zwingend erforderlich.
- Produktbehandlung
  Insbesondere bei Obst wird ein Aufschwimmen des Produkts beobachtet. Damit kann keine ausreichende Behandlung mit Spannungsimpulsen erreicht werden.
- Wirkung des elektrischen Feldes

Zur Optimierung des erforderlichen Energieeintrags ist es erforderlich, dass das Produkt gegenüber dem gepulsten elektrischen Feld eine Relativbewegung ausführt. Es ist also eine kontinuierliche Förderung erforderlich.

Daraus stellte sich die Aufgabe die zu der Erfindung führte, nämlich eine Anlage bereitzustellen, die es ermöglicht, bei relativ geringen Förderquerschnitten hohe Masseströme, z. B 600 Mg/h, kontinuierlich durch ein periodisch oder in vorgegebenen Zeitabständen gepulstes elektrisches Feld zu fördern. In Kombination mit geeigneten Impulserzeugungseinrichtungen, z.B. einer Kondensatorbank mit einem gesteuerten oder im Selbstdurchbruch betriebenen Schalter, einem Marxgenerator, ist es möglich, einen nichtthermischen Zellaufschluss, durch irreversible Perforation der Zellmembrane von vegetativen Zellen, bei geringem spezifischem Energiebedarf großtechnisch durchzuführen.

Die Lösung der Aufgabe wird durch einen Elektroporationsreaktor, gemäß den Merkmalen des Anspruchs 1 gelöst.

Der Elektroporationsreaktor besteht aus einer kreiszylindrischen metallischen Trommel 7 mit dielektrischem Überzug oder einer solchen ganz aus dielektrischem Material, die um ihre horizontal liegende zylinderachse/ Rotationsachse mit 0,5 bis 4 Umdrehungen pro min umlaufen kann. Sie hat Mitnehmer 5 auf der äußeren Mantelwand der Trommel 7, die darauf gleichverteilt um den Umfang angebracht sind.

Der Elektroporationsreaktor hat ein Gehäuse 11 aus dielektrischem Material, das mit seiner Reaktorwand 12 die Trommel 7 mit ihren Mitnehmern 5 bis auf einen oberhalb der Rotationsachse 3 liegenden offenen Bereich berührungslos aber äquidistant umgibt. Die Mitnehmer sitzen parallel zur Rotationsachse der Trommel und sind radial nach außen ausgerichtet. Eine doppelwandige Kammer aus dielektrischem Material, umgibt mit ihrer Innenwand die Trommel mit ihren Mitnehmern bis auf einen oberhalb der Drehachse liegenden offenen Bereich berührungslos und äquidistant. Der Reaktor hat eine Prozessgut-Aufgabevorrichtung 13, an deren unteren Bereich ein Zuführrechen 6 angebaut ist, der an der Zuführzone a des Elektroporationsreaktors angebracht ist und durch den die Mitnehmer 5 der Trommel 7 laufen. Die Prozessgutaufgabevorrichtung mündet in den offenen Bereich der Kammer und dockt mit ihrem oben liegenden Bereich ihrer Mündung an der Innenwand der Kammer am oberen Rand des offen liegenden Bereichs an. Am unten liegenden Bereich der Mündung ist ein Zuführrechen angebaut, durch den die Mitnehmer der Trommel laufen. Eine Austragungsschurre setzt am untenliegenden offenen Bereich der Kammer an der Innenwand des Elektroporationsreaktors an. Ein Austragsrechen, durch den die Mitnehmer der Trommel nach dem Auftauchen aus der Prozessflüssigkeit ebenfalls laufen, sammelt das heran geförderte, inzwischen elektrisch prozessierte Prozessgut auf und lenkt es auf die Austragsschurre zum Weitertransport.

Eine Entgasungszone b und eine Reaktionszone c bestehen im Spalt zwischen der Trommel 7 und der Reaktorwand 12 des Gehäuses 11 sowie eine Austragszone d, die von den Mitnahmefinger 5 durchlaufen werden.

Im tiefstliegenden Bereich der Reaktorwand 12 sitzt mindestens eine zur Trommel 7 hin blank liegenden Elektrodengruppe 1 aus mindestens einer Elektrode 1, die sich höchstens über die Länge des Trommelmantels erstreckt und stets vollständig von der Prozessflüssigkeit benetzt ist. Jede Elektrodengruppe 1 ist über einen hochspannungsgeeigneten, gesteuerten oder im Selbstdurchbruch betriebenen, schnellen Schalter an einen externen elektrischen Energiespeicher, der für den Zweck hinreichend schnell an die Elektrodengruppe gelegt werden kann, nur an diese Elektrodengruppe angeschlossen.

Es bestehen Gruppen von Öffnungen 2 zum Spalt hin im dielektrischen Überzug auf der metallischen Trommel 7 oder Gruppen von zum Spalt hin blank liegenden geerdeten Elektroden darauf oder auf der Trommel 7 aus dielektrischem Material. Die blank liegenden Elektrodenflächen sind auf der Trommel 7 über die Trommelachse 3 geerdet, um im Spaltbereich der mit Hochspannung zu beaufschlagenden Elektrodengruppe innerhalb von höchstens 3 µsec eine elektrische Feldstärke von 10 kV/cm aufbauen zu können, damit das mitgenommene, sich in der Prozessflüssigkeit befindliche Prozessgut an seinen biologischen Zellen entlang deren jeweiliger Hauptachse z, längere Achse einer biologischen Zelle, mindestens einmal beim Durchgang die Schwellenpotentialdifferenz $\Delta\varphi = z * E = 10$ V für irreversible Elektroporation erreicht.

Im Spaltbereich besteht eine Prozess-Flüssigkeitsfüllung mit einem Pegel unterhalb der Drehachse 3 und oberhalb der höchstliegenden Elektrodengruppe. Es besteht weiter eine Tiefe des Eintauchbereiches des Prozessgutes in die Prozessflüssigkeit, die Entgasungszone b, über mindestens eine Spaltbreite zur sichern Entlüftung des Gemisches aus Prozessgut und Prozessflüssigkeit.

In den Unteransprüchen sind weitere Eigenschaften beschrieben, die Vorteile für den zu betreibenden Elektroporationsreaktor sind: Nach Anspruch 2 haben die an der Trommel 7 angebrachten Mitnehmer 5 einen dielektrischen Überzug oder sind aus einem dielektrischen Material, sie sind biegesteif und an ihrer exponierten Oberfläche abriebfest und inert gegen die Prozessumgebung. Die an der Trommel angebrachten biegesteifen Mitnehmer 5 sind elastisch gelagert. Nach Anspruch 4 schließlich ist der Elektroporationsreaktor durch eine metallische Umhüllung nach außen hin elektromagnetisch dicht.

Innerhalb vorgebbarer Zeitabstände wird jeweils ein hohes elektrisches Potential an diese Elektrodengruppe gelegt, wodurch sich zu der auf der Trommel montierten Potentialelektroden hin, die über die Trommelachse geerdet ist, ein möglichst homogenes elektrisches Feld ausbildet, das stets so stark ist, dass das mitgenommene, sich in der Prozessflüssigkeit befindliche Prozessgut elektroporiert wird.

Bei der betriebsbereiten Anlage ist die zum Mantel der Trommel hin exponierte Fläche jeder Elektrodengruppe stets vollständig von der Prozessflüssigkeit benetzt. Auch ist jede Elektrodengruppe über einen eigenen Schalter an einen eigenen elektrischen Energiespeicher angeschlossen. Ein solcher Energiespeicher ist meist eine schnell entladbare Kondensatorbank, um den elektrischen Feld- bzw. Spannungsanstieg in den Reaktionsbereichen stets hinreichend schnell zu schaffen. Hierfür sind etwa Marxgeneratoren gut geeignet.

Weitere Merkmale, die einerseits zweckmäßig sind und andrerseits einen Betrieb mit gutem konstanten Langzeitverhalten ermöglichen, sind:

So ist es erforderlich, das Prozessgut mit geringer Rotationsgeschwindigkeit zwangsweise zu fördern und im Eintauchbereich (Entgasungszone) zu entlüften. Im Bereich der Hochspannungsbehandlung (Reaktionszone) erfährt das während der Bepulsung aufgebaute elektrische Feld durch die Relativbewegung der Elektroden unterschiedliche Ausrichtungen, was zu einer deutlichen Verbesserung des Behandlungserfolgs führt.

Während des Betriebs steht der Pegel der Prozessflüssigkeit stets zwischen der Rotationsachse der Trommel und den am höchsten sitzenden Pulselektroden bzw. Elektrodengruppen. Der Eintauchbereich in die Prozessflüssigkeit ist zur sicheren Entlüftung und damit Befreiung von Luftblasen des Gemisches aus Prozessgut und Prozessflüssigkeit elektrodenfrei gehalten.

Die gesamte Anlage ist gegenüber der Umgebung elektromagnetisch abgeschirmt, um Störungen an Gerät außerhalb liegenden Geräten und Einrichtungen nicht eintreten zu lassen.

Die Erfindung wird in ihrer Funktion und ihrem Aufbau im folgenden anhand der Zeichnung noch näher erläutert. Die Zeichnung besteht aus den Figuren 1 bis 3. Sie zeigen im einzelnen:

Figur 1 Schnitt Seitenansicht des Elektroporationsreaktors

Figur 2 Axialer Schnitt durch den Elektroporationsreaktor.

Figur 3 Abwicklung der Mantelflächen des Reaktionsraums mit Elektrodenanordnung.

Die folgende Beschreibung erfolgt am Beispiel der Behandlung von Rüben:

Die zuvor gewaschenen Rüben gelangen über die Produktaufgabevorrichtung 13 und den Zuführrechen 6 in die Zuführzone a des Elektroporationsreaktors und kommen in der Förderkammer zum Liegen. Die Förderkammer oder der Spalt mit Reaktionszone wird durch die Trommel 7 mit hier einem dielektrischem Überzug und die äußere Begrenzung der Reaktionskammer 12 gebildet.

Durch das Drehen der Trommel über die Antriebseinheit 4 streifen die Mitnehmer 5 die Rüben vom Zuführrechen 6 ab und ziehen diese in den Förderspalt zwischen Trommel 7 und äußerer Begrenzung 12 der Reaktionskammer. Die zunächst noch trocken geförderten Rüben gelangen nach ca. einer ¼ Umdrehung der Trommel 7 in die Prozessflüssigkeitsvorlage, hier Wasser, des Elektroporationsreaktors. Den Eintauchbereich bildet die Entgasungszone b. Hier wird durch geeignete Maßnahmen wie Wassereindüsung, Vibration oder sonst dafür geeignete Maßnahmen, anhaftende Luftblasen und Luftblasen überhaupt, entfernt. Dies ist wichtig, da die beim Durchschlagen der Hochspannung an Gasblasen Schockwellen entstehen, die den Reaktor allmählich, also länger- oder langzeitlich in seiner Funktion beeinträchtigen, ihn sogar zerstören.

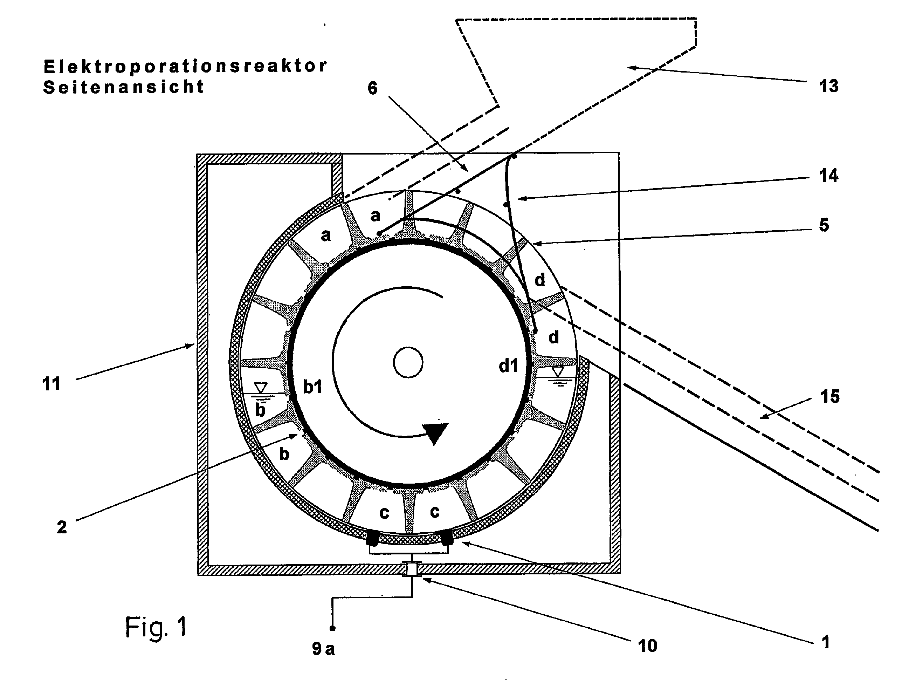

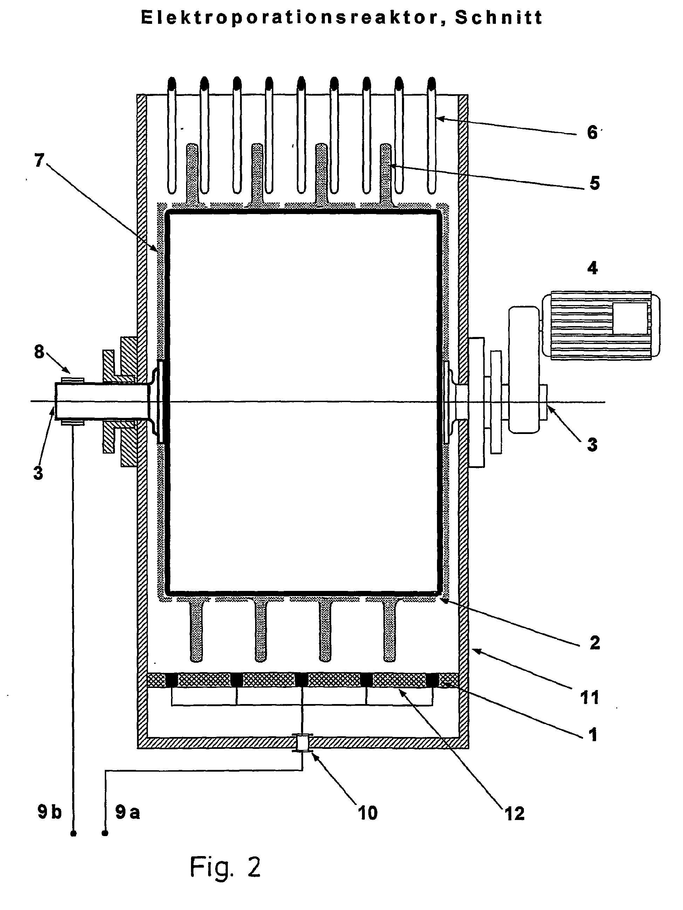

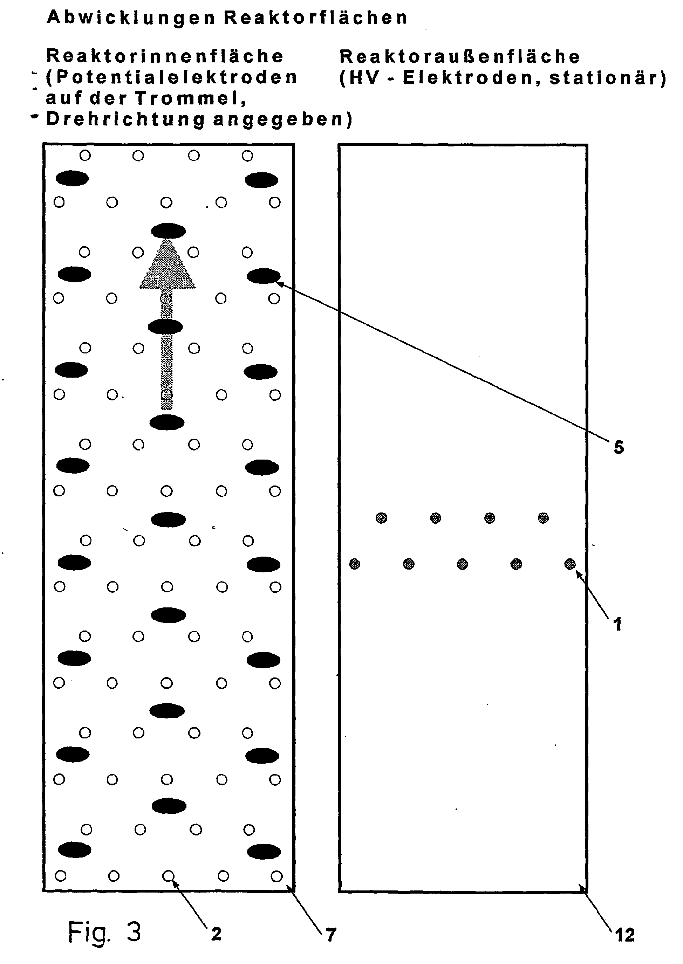

Nach dem Eintauchen in die Wasservorlage und der Entgasung werden die Rüben sukzessive in die Reaktionszonen c gefördert. Das sind gemäß Figur 1 zwei, kann auch nur eine sein, können aber auch mehr als zwei sein. Die Pulsspannung, hier bis zu einigen 100 kV, wird über die metallischen Elektroden 1 in das Wasser eingekoppelt. Die mit Hochspannung beaufschlagbaren Elektroden 1 sind hier in der hochspannungsisolierenden Wand der Kammer 12 ohne Überhöhung zur Trommel hin eingebaut (siehe Figuren 1, 2 und 3). Die für die Hochspannungsentladung erforderlichen Gegenelektroden 2 bzw. das Bezugs- oder Erdpotential sind in der Mantelfläche der Trommel 7 durch das in den Spalt exponierte blanke Metall hergestellt (siehe die Abwicklung in Figur 3). Durch den Winkelversatz der einzelnen Pulselektroden erfährt das elektrische Feld ebenfalls unterschiedliche Ausrichtungen.

Nach dem Weiterdrehen der Förderkammern heben die Mitnehmer die prozessierten Rüben aus dem Wasserbad, sie werden dann über den Austragsrechen 14 aus der Förderkammer abgestreift. Hierbei kann das Rübenmaterial abtropfen und wird über die Austragsschurre 15 der weiteren Verarbeitung zugeführt.

Mitnehmer 5, Reaktorgehäuse 11 im Bereich der Reaktionskammer, dielektrische Isolationsschicht der Trommel 7 sowie Hochspannungsisolation der Pulselektroden 12 sind aus elektrisch isolierendem Werkstoff wie Polyethylen natur, Polyethylen schwarz, Polypropylen grau, Polyurethan PU, verstärktes bzw. glasfaserverstärktem Werkstoffen hergestellt, bzw. durch dieses elektrisch isoliert.

Form und Oberfläche der Mitnehmer 5 ist so optimiert, dass die erforderliche mechanische Stabilität vorhanden ist und Hochspannungsgleitentladungen verhindert werden.

Zur Unterdrückung von elektromagnetischer Strahlung in die Umgebung ist die Anlage ausreichend, z.B. metallisch geschirmt.

Die oberhalb des Flüssigkeitsspiegels liegende Drehachse/Welle 3 der Trommel vermeidet nicht einfach zu beherrschende Abdicht- und damit elektrische Isolationsprobleme.

Bezugszeichenliste

1 Elektrode, Elektrodengruppe
2 Bezugspotential- bzw. Erdpotentialelektrode
3 Rotationsachse, Welle
4 Antriebseinheit, Motor
5 Mitnehmer
6 Zuführrechen
7 Trommel
8 Potentialabgriff
9a Potentialanschluss Impulserzeugung
9b Pulsanschluss Impulserzeugung
10 Hochspannungsdurchführung
11 Reaktorgehäuse
12 Reaktionskammer
13 Produktaufgabevorrichtung
14 Austragsrechen
15 Austragsschurre

a Zuführzone
b Entgasungszone
b1 Füllstand Prozessflüssigkeit Ablauf
c Reaktionszone, wirksamer Bereich kritische elektrisches Feld
d1 Füllstand Prozessflüssigkeit Zulauf
d Austragszone

**Patentansprüche**

1. Elektroporationsreaktor zur kontinuierlichen Prozessierung von stückigen Produkten, dem Prozessgut, wie Agrarprodukten, wie Zuckerrüben, Kartoffel, Gemüse, Obst, Heilpflanzen und auch tierische Produkte, in einer Prozessflüssigkeit mit Hochspannungsimpulsen
bestehend aus:

einer kreiszylindrischen metallischen Trommel (7) mit dielektrischem Überzug oder ganz aus dielektrischem Material, die um ihre horizontal liegende Zylinderachse/ Rotationsachse mit 0,5 bis 4 Umdrehungen pro min umlaufen kann,
Mitnehmern (5) auf der äußeren Mantelwand der Trommel (7), die darauf gleich verteilt um den Umfang angebracht sind,
einem Gehäuse (11) aus dielektrischem Material, das mit seiner Reaktorwand (12) die Trommel (7) mit ihren Mitnehmern (5) bis auf einen oberhalb der Rotationsachse (3) liegenden offenen Bereich berührungslos aber äquidistant umgibt,
einer Prozessgut-Aufgabevorrichtung (13), an deren unterem Bereich ein Zuführrechen (6) angebaut ist, der an der Zuführzone (a) des Elektroporationsreaktors angebracht ist und durch den die Mitnehmer (5) der Trommel (7) laufen,
einer Entgasungszone (b), einer Reaktionszone (c) im Spalt zwischen der Trommel (7) und der Reaktorwand (12) des Gehäuses (11) sowie einer Austragszone (d), die von den Mitnahmefingern (5) durchlaufen werden,
einem Austragsrechen (14), durch den die Mitnehmer (5) der Trommel (7) laufen, um das herangebrachte, inzwischen prozessierte Prozessgut aufzusammeln und auf einer an der Austragszone (d) ansetzenden Austragsschurre (15) zu lenken, mindestens einer zur Trommel (7) hin blank liegenden Elektrodengruppe (1) aus mindestens einer Elektrode (1) im tiefstliegenden Bereich der Reaktorwand (12), die sich höchstens über die Länge des Trommelmantels erstreckt und stets vollständig von der Prozessflüssigkeit benetzt ist und jede Elektrodengruppe über einen eigenen Schalter an einen eigenen elektrischen Energiespeicher angeschlossen ist, und
Gruppen von Öffnungen (2) im dielektrischen Überzug auf der metallischen Trommel (7) zum Spalt hin oder
Gruppen von zum Spalt hin blank liegenden geerdeten Elektroden darauf oder
auf der Trommel (7) aus dielektrischem Material,
wobei die blank liegenden Elektrodenflächen auf der Trommel (7) über die Trommelachse (3) geerdet sind,
um im Spaltbereich der mit Hochspannung zu beaufschlagenden Elektrodengruppe innerhalb von höchstens 3 μsec eine elektrische Feldstärke von 10 kV/cm einrichten zu können, damit das mitgenommene, sich in der Prozessflüssigkeit befindliche Prozessgut an seinen biologischen Zellen entlang deren jeweiliger Hauptachse z, längere Achse einer biologischen Zelle, mindestens einmal beim Durchgang die Schwellenpotentialdifferenz

$$\Delta\varphi = z * E = 10 \text{ V}$$

für irreversible *Poration der Zellwand,* die Elektroporation, erreicht,
einer Prozess-Flüssigkeitsfüllung im Spalt mit einem Pegel unterhalb der Drehachse (3) und oberhalb der höchstliegenden Elektrodengruppe

einer Tiefe des Eintauchbereiches des Prozessgutes in die Prozessflüssigkeit, die Entgasungszone (b), über mindestens eine Spaltbreite zur sichern Entlüftung des Gemisches aus Prozessgut und Prozessflüssigkeit.

2. Elektroporationsreaktor nach Anspruch 1, **dadurch gekennzeichnet, dass**, die an der Trommel (7) angebrachten Mitnehmer (5) einen di elektrischen Überzug haben oder aus einem dielektrischen Material sind, biegesteif und an ihrer exponierten Oberfläche abriebfest und inert gegen die Prozessumgebung sind.

3. Elektroporationsreaktor nach Anspruch 2, **dadurch gekennzeichnet, dass**, die an der Trommel angebrachten biegesteifen Mitnehmer (5) elastisch gelagert sind.

4. Elektroporationsreaktor nach einem der Ansprüche 1 bis 3, **dadurch gekennzeichnet, dass**, die Anlage durch eine metallische Umhüllung nach außen hin elektromagnetisch dicht ist.

**Claims**

1. Electroporation reactor for the continuous processing of lumpy products, the feedstock, such as agricultural products like sugar beets, potatoes, vegetables, fruits, medical plants, and animal products, in a process fluid with high-voltage pulses, consisting of:

   a cylindrical metal drum (7) with a dielectric coating or entirely made of dielectric material, which rotates around its *horizontal* cylinder axis/rotation axis at 0.5 to 4 rotations per minute, pins (5) on the outer wall of the drum (7), which are arranged homogeneously along the circumference, a housing (11) of dielectric material, the reactor wall (12) of which encloses the drum (7) and its pins (5) in a contact-free, but equidistant manner, except for an open area above the rotation axis (3), a feedstock supply unit (13), to the lower end of which a supply rake (6) is fixed, which is located in the supply zone (a) of the electroporation reactor and passed by the pins (5) of the drum (7), a degasification zone (b), a reaction zone (c) in the gap between the drum (7) and the reactor wall (12) of the housing (11), and a discharge zone (d), all of which are passed by the pin fingers (5), a discharge rake (14) which is passed by the pins (5) of the drum (7) in order to collect the approaching, meanwhile processed feedstock and to direct it to a discharge chute (15) attached to the discharge zone (d), at least one electrode group (1) lying bare towards the drum (7), which consists of at least one electrode (1) in the lowest range of the reactor wall (12), extends over the length of the drum at the maximum, and always is wet completely by the process fluid, with each electrode group being connected to a separate electric energy accumulator via a separate switch, and groups of openings (2) in the dielectric coating on the metal drum (7) directed towards the gap or groups of earthed electrodes lying blank towards the gap and arranged on the coating or on the drum (7) of dielectric material, with the blank electrode surfaces on the drum (7) being earthed via the drum axis (3), to generate an electric field of 10 kV/cm within not more than 3 μsec in the gap range of the electrode group to be loaded by high voltage, such that the feedstock supplied and now located in the process fluid is subjected to electroporation of its biological cells along the respective main axis z, the longer axis of a biological cell, at least once when exceeding the threshold potential difference

$$\Delta\varphi = z * E = 10 \text{ V}$$

for the irreversible *poration* of the *cell wall,* a process fluid level in the gap below the rotation axis (3) and above the highest located electrode group, and a depth of immersion of the feedstock into the process fluid, the degasification zone (b), of at least one gap width for the safe venting of the mixture of feedstock and process fluid.

2. Electroporation reactor according to Claim 1, **characterized by** the pins (5) attached to the drum (7) having a dielectric coating or being made of a dielectric material and being bending-resistant and abrasion-resistant at their exposed surface as well as inert to the process environment.

3. Electroporation reactor according to Claim 2, **characterized by** the bending-resistant pins (5) attached to the drum being provided with an elastic bearing.

4. Electroporation reactor according to one of Claims 1 through 3, **characterized by** the plant being electromagnetically tight to the outside due to a metal

## Revendications

1.  Électroporateur destiné au traitement en continu de matières en morceaux entiers, appelées masse à traiter, tels que de produits agricoles, comme les betteraves sucrières, les pommes de terre, les légumes, les fruits, d'herbes médicinales mais aussi de produits d'origine animale, dans un liquide de traitement soumises à des impulsions haute tension comprenant

    un tambour cylindrique circulaire métallique (7) enrobé d'une matière diélectrique ou réalisé entièrement en une matière diélectrique pouvant tourner autour de son axe cylindrique logé de manière horizontale /son axe de rotation à une vitesse de rotation de 0,5 à 4 tours/minute,

    des doigts d'entraînement (5) disposés sur la paroi extérieure de la chemise du tambour (7), répartis de manière uniforme sur toute la circonférence de ladite paroi,

    un corps (11) réalisé en une matière diélectrique entourant de sa paroi de réacteur (12), sans le toucher mais de manière équidistante, le tambour (7) pourvu de ses doigts d'entraînement (5) jusqu'à une zone ouverte située au-dessus de l'axe de rotation (3),

    un dispositif de chargement de la masse à traiter (13), muni dans sa partie inférieure d'un rateau d'alimentation (6) monté sur la zone d'alimentation (a) de l'électropolariseur et à travers duquel passent les doigts d'entraînement (5) du tambour (7),

    une zone de dégazage (b), une zone de plasmolyse (c) située dans la fente d'entraînement de la matière formée par le tambour (7) et la paroi du réacteur (12) du corps (11), ainsi qu'une zone de déversement (d) parcourue par les doigts d'entraînement (5),

    un rateau de déversement (14), parcouru par les doigts d'entraînement (5) du tambour (7) et destiné à collecter la masse une fois traitée et l'orienter vers une goulotte (15) aboutée à la zone de déversement (d),

    au moins un groupe d'électrodes à nu (1) côté tambour (7) comprenant au moins une électrode (1) dans la partie la plus basse de la paroi du réacteur (12), groupe s'étendant au plus sur la longueur de la chemise du tambour et baignant sans cesse complètement dans le liquide de traitement, chaque groupe d'électrodes étant raccordé à son propre accumulateur d'énergie électrique à l'aide de son propre commutateur
    et

    des groupes d'ouvertures (2) réalisées dans le revêtement diélectrique du tambour métallique (7) et orientées vers la fente ou des groupes d'électrodes à nu (1) mises à la terre du côté de ladite fente disposées sur ledit revêtement diélectrique ou sur le tambour (7) réalisé en matière diélectrique, les surfaces des électrodes à nu disposées sur le tambour étant mises à la terre au travers de l'axe (3) dudit tambour de manière à pouvoir réaliser, en 3 microsecondes au plus, une intensité de champ électrique de 10 kV/cm dans la zone de la fente pourvue de groupes d'électrodes devant être alimentées en courant haute tension de telle sorte que la masse à traiter entraînée par le liquide de traitement dans lequel elle baigne puisse atteindre, au moins une fois par passe un potentiel de seuil aux électrodes de

    $$\Delta\varphi = z * E = 10 \text{ V}$$

    le long chaque axe principal z de ses cellules biologiques, lequel constitue l'axe le plus long d'une cellule biologique, permettant une plasmolyse irréversible de la paroi cellulaire,

    un volume de remplissage de liquide de traitement dans la fente dont le niveau se situe en-deça de l'axe de rotation (3) mais au-dessus du groupe d'électrodes le plus haut,

    une profondeur de plongée de la matière à traiter dans le liquide de traitement constituant zone de dégazage (b) égale au moins à une largeur de fente afin d'autoriser une ventilation sûre du mélange constitué de la masse à traiter et du liquide de traitement.

2.  Électroporateur selon la revendication 1, **caractérisé en ce que** les doigts d'entraînement (5) fixés sur le tambour (7) présentent un revêtement diélectrique ou sont réalisés en une matière diélectrique, sont résistants à la flexion et à l'abrasion au niveau de leurs surfaces exposées et inertes par rapport au milieu de traitement.

3.  Électroporateur selon la revendication 2, **caractérisé en ce que** les doigts d'entraînement (5) fixés sur le tambour sont montés élastiques.

4.  Électroporateur selon l'une quelconque des revendications 1 à 3, **caractérisé en ce que** vers l'extérieur l'installation est rendu étanche aux rayonnements électromagnétiques au moyen d'une enveloppe métallique.

Elektroporationsreaktor
Seitenansicht

Fig. 1

EP 1 424 910 B1

# Elektroporationsreaktor, Schnitt

Fig. 2

**Abwicklungen Reaktorflächen**

**Reaktorinnenfläche**
**(Potentialelektroden**
**auf der Trommel,**
**Drehrichtung angegeben)**

**Reaktoraußenfläche**
**(HV - Elektroden, stationär)**

Fig. 3    2    7    12